# DEMANDE DE BREVET EUROPEEN

(11) **EP 0 824 013 A1**
(43) Date de publication de la demande: **18.02.1998**
(21) Numéro de dépôt: 96440060.0
(22) Date de dépôt: 13.08.1996
(51) Int. Cl.: A61F 2/36, A61B 17/72

(54) **Prothèse fémorale modulaire à géométrie variable, notamment de reconstruction**

(71) Demandeur: SOCIETE CIVILE SOIRIE, 01150 Chazey sur Ain (FR)
(72) Inventeur: CHATELET, Jean-Christophe, F-01150 CHAZEY SUR AIN (FR); FISCHER, Louis, F-69160 TASSIN LA DEMI-LUNE (FR); FESSY, Michel-Henri, F-69390 MILLERY (FR)
(74) Mandataire: Dénoyez, Hubert

(57) **Abrégé**

Prothèse fémorale modulaire à géométrie variable, notamment de reconstruction.

Elle comprend deux parties distinctes adaptables et solidarisables l'une à l'autre au moyen d'une vis de serrage (3), d'une part un clou diaphysaire (1) et d'autre part une partie métaphysaire (2) ; le clou diaphysaire (1) comportant deux parties, une partie supérieure cylindrique ou tronconique (10) de solidarisation à la partie métaphysaire (2) et une partie inférieure (11) courbe, dont l'extrémité est fendue dans le plan frontal et dont la courbure permet son adaptation anatomique, destinée à être introduite dans le canal médullaire du fémur; la partie métaphysaire (2) comportant axialement un canal (23) destiné à abriter ladite partie supérieure cylindrique ou tronconique (10) du clou diaphysaire (1).

## Description

La présente invention a pour objet une prothèse de hanche modulaire à géométrie variable, et plus particulièrement une prothèse fémorale, notamment de reconstruction en remplacement d'une prothèse implantée antérieurement.

Il existe a ce jour de nombreux types de prothèses totales de hanche, qui comportent une prothèse fémorale implantée dans le fémur par impaction ou cimentation, et un cotyle implanté dans l'os du bassin.

Or il arrive assez fréquemment que la prothèse fémorale se désolidarise sensiblement de l'os du fémur, notamment dans le cas de prothèses fémorales des générations antérieures. Il est alors nécessaire de l'enlever et de la remplacer par une prothèse de reprise ou de reconstruction.

Dans certains cas de remplacement, comme lors de remplacements successifs, il est fréquent que ces reprises entraînent, à terme, une destruction de la zone proximale du fémur, qu'il faut alors reconstruire par une prothèse de reconstruction fémorale.

Toutefois la prothèse fémorale ne peut être fixée a l'os dans sa zone proximale puisque cette zone, endommagée, ne peut permettre un support suffisamment solide. Il est donc nécessaire de pouvoir fixer ce type de prothèse le plus bas possible, dans la zone où l'os fémoral est encore sain et où la fixation peut encore être assurée.

Or, pour descendre le plus bas possible à l'intérieur du fémur, il faut respecter la courbure de celui-ci et donc employer des tiges courbes. Mais tous les fémurs ne sont pas strictement identiques et lorsque la prothèse vient se loger dans le canal médullaire, elle prend souvent une orientation qui influe nécessairement sur la position, antéversée ou rétroversée, du col prothètique.

La présente invention a pour but de proposer une prothèse fémorale de reconstruction modulaire à géométrie variable, susceptible d'être adaptée à chaque cas particulier tout en étant d'une mise en place aisée et d'une solidité accrue.

Une prothèse fémorale de reconstruction modulaire selon l'invention se caractérise essentiellement en ce qu'elle comporte deux parties complémentaires adaptables l'une à l'autre, à savoir un clou diaphysaire et une partie métaphysaire, et en ce que le clou diaphysaire, qui présente une courbure permettant son adaptation anatomique, est percé transversalement, dans sa partie distale, d'au moins deux orifices permettant sa fixation au moyen de vis, son extrémité distale étant en forme d'ogive et étant fendue dans le sens de la courbure, tandis que son extrémité proximale est crantée extérieurement et est taraudée axialement pour permettre la fixation, au moyen d'une vis, de la partie métaphysaire ; et en ce que ladite partie métaphysaire comporte centralement un canal divisé en plusieurs zones, une zone inférieure lisse dans laquelle se loge l'extrémité proximale du clou diaphysaire, une zone intermédiaire cylindrique cannelée permettant, en association avec l'extrémité proximale crantée du clou diaphysaire, l'ajustement angulaire de ladite partie métaphysaire par rapport au clou diaphysaire, et une zone supérieure tronconique permettant le blocage par compression de la vis de fixation, l'extrémité supérieure de ladite zone tronconique étant lamée de manière à former une chambre destinée à abriter la tête de la vis de fixation, laquelle est recouverte d'un capuchon de protection qui s'encliquette dans ladite chambre.

Selon une caractéristique additionnelle de la prothèse selon l'invention la partie inférieure lisse du canal de la partie métaphysaire est tronconique à faible pente, de même que la partie inférieure de la zone lisse du clou diaphysaire.

Dans un mode de réalisation préférentiel de la prothèse* selon l'invention, la partie métaphysaire est recouverte extérieurement d'hydroxyapatite permettant sa fixation biologique.

Afin d'obtenir la forme la mieux adaptée à l'anatomie du patient, il convient de disposer de clous diaphysaires de longueurs et de diamètres différents, et de parties métaphysaires de tailles différentes, adaptables les uns aux autres.

Les avantages et les caractéristiques de la présente inventi on ressortiront plus clairement de la description qui suit et qui se rapporte au dessin annexé, lequel en représente un mode de réalisation non limitatif.

Dans le dessin annexe :
- la figure 1 représente une vue de face et en éclaté d'une prothèse fémorale de reconstruction selon l'invention.
- la figure 2 représente une vue de profil, avec arrachement partiel, de la partie métaphysaire de la même prothèse.
- la figure 3 représente une vue à plus grande échelle de la même partie métaphysaire, en coupe transversale selon l'axe XX' porté sur la figure 2.
- la figure 4 représente une vue de profil, avec arraché partiel, de l'extrémité proximale du clou diaphysaire de la même prothèse.
- la figure 5 représente une vue en plan de l'extrémité proximale du même clou diaphysaire.

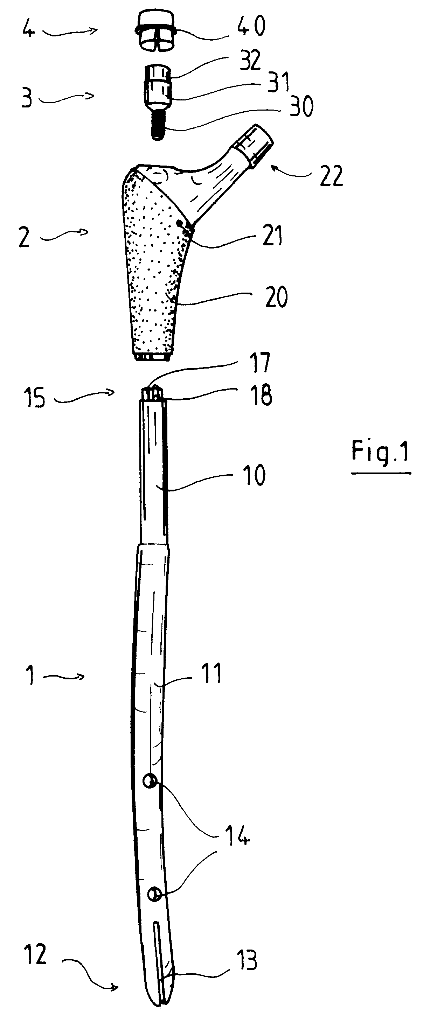

Si on se réfère à la figure 1 on peut voir qu'une prothèse fémorale de reconstruction selon l'invention est essentiellement constituée d'un clou diaphysaire 1, d'une partie métaphysaire 2, d'une vis de fixation 3 et d'un capuchon protecteur 4.

La partie métaphysaire 2 est de forme classique, et son corps 20 est recouvert d'hydroxyapatite permettant sa fixation biologique.

Un orifice 21 est percé transversalement dans l'extrémité supérieure interne du corps 20 afin de permettre une éventuelle ligature du grand trochanter et des muscles fessiers.

Le col 22, de façon connue en soi, est tronconique et permet la solidarisation d'une tête fémorale, non représentée.

Le clou diaphysaire 1 comporte deux parties distinctes, une partie supérieure cylindrique 10 de solidarisation à la partie métaphysaire 2 et une partie inférieure 11 destinée à être introduite dans le fémur, et présentant une courbure qui lui permet de s'adapter au mieux à l'anatomie.

L'extrémité distale 12 du clou diaphysaire 1 comporte une fente longitudinale 13 dans le sens de la courbure, afin d'éviter les pics de contraintes.

Deux orifices 14 sont percés transversalement dans la partie inférieure courbe 11 afin de permettre la fixation du clou diaphysaire 1 dans le fémur.

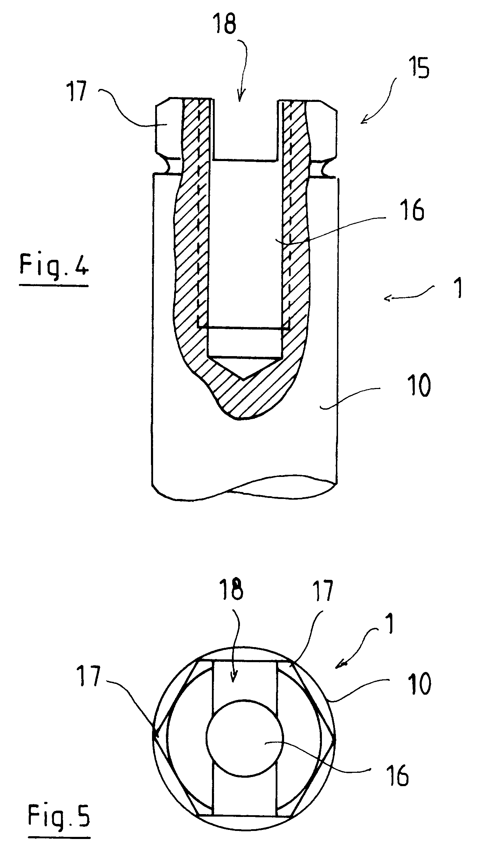

Si on se réfère également aux figures 4 et 5 on peut voir que l'extrémité proximale 15 du clou diaphysaire 1 comporte d'une part un orifice axial taraudé 16 permettant le vissage de la vis 3, d'autre part extérieurement des crans 17, et d'autre part encore une fente transversale 18 permettant l'adaptation d'un gabarit de perçage, non représenté, utilisé pour pratiquer dans le fémur des perçages transversaux en regard des orifices 14, en vue d'y insérer des vis.

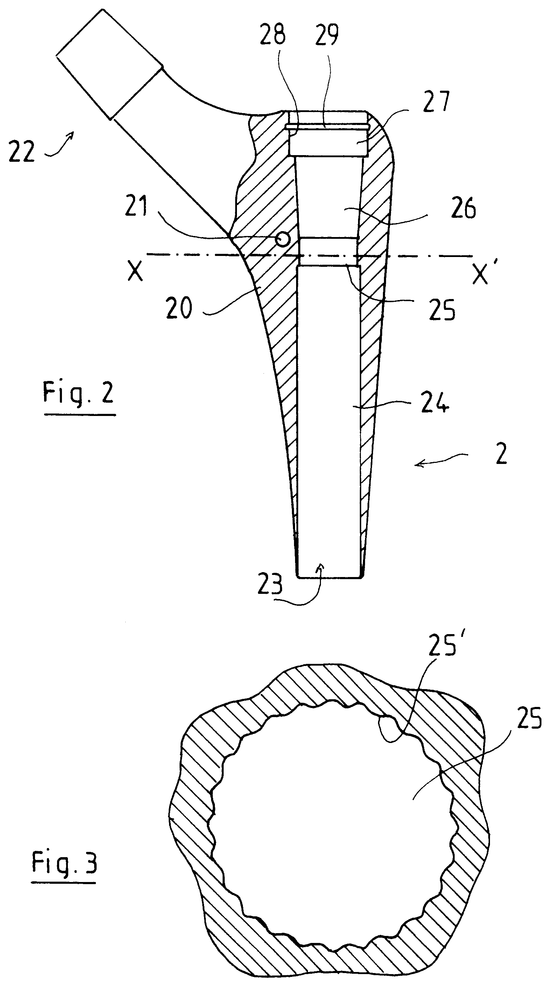

Si on se réfère maintenant aux figure 2 et 3 on peut voir que la partie métaphysaire 2 comporte centralement et axialement un canal 23 divisé en plusieurs zones, une zone inférieure 24 cylindrique dans laquelle est logée la partie cylindrique 10 du clou diaphysaire 1, une zone intermédiaire 25 cylindrique comportant des cannelures longitudinales 25', dans laquelle est logée l'extrémité proximale 15 du clou diaphysaire et permettant des réglages angulaires, et une zone tronconique 26 autorisant le serrage de la vis 3.

Il y a lieu de noter que la base de la partie 10 du clou diaphysaire 1 et la zone inférieure 24 du canal 23 peuvent être de formes tronconiques complémentaires afin de parfaire le blocage.

Les crans 17 sont au nombre de 6 et possèdent une légère pente de 2° pour faciliter leur introduction dans les cannelures 25', qui au nombre de 24 permettent un réglage angulaire de 15° en 15°, étant bien entendu que d'autres valeurs peuvent être retenues.

La vis 3 comporte trois parties, une tige filetée 30 surmontée d'une partie tronconique 31, elle-même surmontée d'une tête 32 à empreinte creuse non visible sur la figure, la tête 32 étant d'un diamètre inférieur à celui de la partie tronconique 31.

Une chambre 27 est pratiquée à l'extrémité du canal 23 de la partie métaphysaire 2 afin d'abriter la tête 32 de la vis 3, la paroi 28 de cette chambre 27 comportant une gorge périphérique 29 dans laquelle s'encliquette la saillie périphérique 40 du capuchon 4 lorsque celui-ci est introduit dans la chambre 27 pour recouvrir la tête 32 de la vis 3.

La pose d'une prothèse fémorale de reconstruction selon l'invention se déroule de la façon suivante:
- le clou diaphysaire 1 est mis en place, sa forme courbe lui permettant de s'orienter de lui-même, puis il est verrouillé par introduction de vis dans les orifices 14.
- on réalise dans le fémur un évasement métaphysaire et l'on pose des greffons, en utilisant à cet effet une partie métaphysaire d'essai, l'antéversion étant choisie par le chirurgien pendant la pose des greffons.
- on adapte la partie métaphysaire 2 au clou diaphysaire 1 par impaction, puis on les solidarise au moyen de la vis 3 que l'on recouvre du capuchon 4, après quoi il ne reste plus qu'à adapter la tête fémorale.

Il va de soi que la présente invention ne saurait être limitée a la description qui précède d'un de ses modes de réalisation, susceptible de subir un certain nombre de modifications sans pour autant sortir du cadre de l'invention.

## Revendications

1. Prothèse fémorale modulaire à géométrie variable, notamment de reconstruction, caractérisée en ce qu'elle comprend deux parties distinctes adaptables et solidarisables l'une à l'autre au moyen d'une vis de serrage (3), d'une part un clou diaphysaire (1) et d'autre part une partie métaphysaire (2); le clou diaphysaire (1) comportant deux parties, une partie supérieure cylindrique ou tronconique (10) de solidarisation à la partie métaphysaire (2) et une partie inférieure (11) courbe, dont l'extrémité est fendue dans le plan frontal et dont la courbure permet son adaptation anatomique, destinée à être introduite dans le canal médullaire du fémur; la partie métaphysaire (2) comportant axialement un canal (23) destiné à abriter ladite partie supérieure cylindrique ou tronconique (10) du clou diaphysaire (1), laquelle comporte axialement, à son extrémité, un orifice taraudé (16) dans lequel est vissée ladite vis (3).

2. Prothèse selon la revendication 1 caractérisée en ce que l'extrémité proximale (15) du clou diaphysaire (1) comporte extérieurement des crans (17) aptes à s'insérer, selon des angles différents, dans une zone intermédiaire cannelée (25) du canal (23) de la partie métaphysaire (2).

3. Prothèse selon la revendication 1 ou la revendication 2 caractérisée en ce que le canal (23) de la partie métaphysaire (2) comporte dans sa partie supérieure une zone tronconique (26) permettant un blocage par compression, par serrage de la vis (3) dans l'orifice taraudé (16) du clou diaphysaire (1).

4. Prothèse selon la revendication 3 caractérisée en ce que l'extrémité supérieure du canal (23) de la partie métaphysaire (2) comporte une chambre (27) destinée à abriter la tête (32) de la vis (3) et permettant l'encliquetage d'un capuchon (4) de protection de celle-ci par introduction d'une saillie périphérique (40) solidaire dudit capuchon (4) dans une gorge périphérique (29) pratiquée dans la paroi (28) de ladite chambre (27).

5. Prothèse selon l'une quelconque des revendications précédentes caractérisée en ce que la partie inférieure courbe (11) du clou diaphysaire (1) comporte au moins deux orifices transversaux (14) permettant la fixation par vis dudit clou diaphysaire (1) dans le fémur.

6. Prothèse selon la revendication 5 caractérisée en ce que l'extrémité proximale (15) du clou diaphysaire (1) comporte une fente transversale (18) permettant l'adaptation d'un gabarit de perçage en vue de pratiquer dans le fémur des perçages en regard des orifices transversaux (14) du chou diaphysaire (1).

7. Prothèse selon l'une quelconque des revendications précédentes caractérisée en ce que la base de la partie lisse (10) du clou diaphysaire (1) et la partie inférieure du canal (23) de la partie métaphysaire (2) sont de formes tronconiques complémentaires.
